# EUROPEAN PATENT APPLICATION

(11) **EP 1 947 188 A1**
(43) Date of publication of application: **23.07.2008**
(21) Application number: 08250173.5
(22) Date of filing: 14.01.2008
(51) Int. Cl.: C12N 15/85

(54) **Methods of inducing and treating vascular disease**

(30) Priority: 16.01.2007 US 885155 P; 12.12.2007 US 954381
(71) Applicant: Cordis Corporation, Miami Lakes, FL 33014 (US)
(72) Inventor: Falotico, Robert, Belle Mead, NJ 08502 (US); Zhao, Jonathon Z., Belle Mead, NJ 08502 (US); Zhao, Lei., Chester Springs, PA 19425 (US)
(74) Representative: Belcher, Simon James

(57) **Abstract**

Animal testing, wherein the animals have disease state characteristics substantially equivalent to those found in humans, may be utilized to evaluate the efficacy of medical devices and/or therapeutic agents.

In one embodiment, a method of inducing one or more vascular diseases in a mammal having at least one of an apoE gene and LDL-receptor gene deleted is described. The method comprises: accelerating athereosclorosis in the mammalian vasculature through the introduction of a high fat, high cholesterol diet; and introducing, via systemic delivery, angiotensin II at a predetermined time to induce and accelerate vascular inflammation.

## Description

The present invention relates to medical test procedures, and more particularly to methods of inducing and treating vascular disease in mammals.

Determining the efficacy of medical devices and/or therapeutic agents initially requires animal testing, wherein the animals preferably have disease state characteristics identical to or approximate the disease state characteristics found in humans. Human disease state characteristics are not typically found in animals. Accordingly, these disease state characteristics may be induced in the animals by various means, including gene manipulation, diet, drug therapy and/or various combinations thereof. The precise control of these inducement means allows researchers the opportunity to approximate many human disease state characteristics.

In accordance with one aspect, the present invention is directed to a method of inducing one or more predetermined vascular diseases in a mammal having at least one of an apoE gene and LDL-receptor gene deleted. The method comprises accelerating athereosclerosis in the mammalian vasculature through the introduction of a high fat, high cholesterol diet and introducing, via systemic delivery, angiotensin II at a predetermined time to induce and accelerate vascular inflammation.

The foregoing and other features and advantages of the invention will be apparent from the following, more particular description of preferred embodiments of the invention, as illustrated in the accompanying drawings.

Embodiments of the invention will now be described by way of example with reference to the accompanying drawings, in which:
Figure 1 is a tabular representation of sirolimus pharmacokinetics in *apoE*^{-/-} mice in accordance with the present invention.
Figure 2 is a tabular representation of body weight, plasma total cholesterol, triglycerides, LDL cholesterol and HDL cholesterol in *apoE*^{*-*/}*⁻* mice in accordance with the present invention.
Figure 3 is a graphical representation of re-suturing incidence post surgery in *apoE*^{-/-} mice in accordance with the present invention.
Figure 4A are first images of aortas of *apoE*^{*-*/}*⁻* mice in accordance with the present invention.
Figure 4B is a graphical representation of percentage lesion area to total aorta area in *apoE*^{*-*/}*⁻* mice in accordance with the present invention.
Figure 5A are second images of aortas of *apoE*^{*-*/}*⁻* mice in accordance with the present invention.
Figure 5B is a graphical representation of quantitative analysis of oil red O positive staining area at the aortic root of *apoE*^{-/-} mice in accordance with the present invention.
Figure 6A are third images of aortas of *apoE*^{*-*/}*⁻* mice in accordance with the present invention.
Figure 6B is a graphical representation of quantitative analysis of CD68 position staining at the aortic root of *apoE*^{*-*/}*⁻* mice in accordance with the present invention.
Figure 7A are third images of aortas of *apoE*^{-/-} mice in accordance with the present invention.
Figure 7B is a graphical representation of the severity of abdominal aortic adventitia lesions in *apoE*^{*-*/}*⁻* mice in accordance with the present invention.
Figures 8A-8D are histological and immunohistochemical images of adventitia lesions in *apoE*^{-/-} mice in accordance with the present invention.

The present invention is directed to a model for creating disease states and determining the efficacy of treatments for these disease states in mammals. More specifically, in this invention artheriosclerosis and aortic lesions are created utilizing a combination of diet and angll in *apoE*^{*-*/}*⁻* mice. Once the disease states are created, various treatments may be utilized. In this exemplary embodiment, rapamycin is utilized as the treatment modality. For the data generated, it appears that treatment with rapamycin reduces atherosclerotic lesions, retards inflammatory macrophage infiltration which is responsible for decreased foam call formation which in turn leads to reduced lipid deposition, and reduces the incidence of adventitial lesions.

As used herein, rapamycin includes rapamycin, sirolimus, everolimus and all analogs, derivatives and conjugates that bind FKBP12, and other immunophilins and possesses the same pharmacologic properties as rapamycin including inhibition of mTOR.

ApoE^{-/-} mice (backcrossed ten times to the C57BL/6 background) were purchased from The Jackson Laboratory. The mice were kept on a twelve-hour light/ twelve-hour dark regimen upon arrival. All animal experiments were approved by the IACUC of the Consumer & Personal Product Worldwide, in accordance with AAALAC guidelines. Eight week old, male apoE^{-/-} mice were fed a normal mouse-chow diet (RP5001; PMI Feeds Inc., St. Louis, MO) and acclimated for one week before switching to a pro-atherogenic high fat, high cholesterol, diet ("*Western* diet", 21 % fat, 0.2 % cholesterol, diet No. 88137, Harlan-Teklad, Madison, WI) for four weeks.

Upon initiation of the pro-atherogenic diet, Alzet osmotic mini-pumps (Model 2004; ALZA Scientific Products, Mountain View, CA) were implanted into the apoE^{-/-} mice subcutaneously. The mini-pumps were loaded with angII (EMD Biosciences, San Diego, CA) at the delivery rate of 1,000 ng/min/kg for twenty-eight days. The mice were anesthetized using isoflurane for the implantation of the mini-pumps. Small incisions in the lower back of the neck were made and the mini-pumps were inserted into the subcutaneous space of the mice. The incisions were then closed using sterile staplers. Triple antibiotic ointment was applied to each incision post surgery. The mice on the high fat, high cholesterol diet were sacrificed by CO₂ inhalation twenty-eight days post mini-pump implantation as is explained in detail subsequently.

Upon initiation of the pro-atherogenic diet and osmotic mini-pump implantation, mice were randomly assigned to four groups; namely, vehicle control, rapamycin or sirolimus 0.5 mg/kg, sirolimus 1.0 mg/kg and sirolimus 4.0 mg/kg body weight. The defined amount of sirolimus was dissolved in ethanol, brought up in a vehicle comprising 0.2 percent Sodium carboxymethylcellulose and 0.25 percent tween 80) and administrated to mice (*i.p.,* daily).

Sirolimus was quantitated using liquid-liquid extraction and reverse-phase liquid chromatography mass spectrometry (LC-MS/MS). Briefly, 100 µl of mouse whole blood sample underwent protein precipitation with 5 percent Methanol/Zinc sulfate solution containing the internal standard ascomycin. After centrifugation of the resulting solution, the liquid mixture proceeded with liquid-liquid extraction with 1-chlorobutane. The combined organic extract was then blown down to dryness and reconstituted with 50:50 Methanol:20 mM ammonium acetate solution. The samples were analyzed on API-5000 using C18 reverse-phase chromatography in Multiple Reaction Monitoring (MRM) mode using atmospheric pressure chemical ionization (ApCl) approach. The calibration standard curve results for sirolimus in mouse whole blood were acceptable from 0.5 ng/ml to 10,000 ng/ml with correlation coefficient of 0.998. The percent bias for the calibration standards ranged from about 10.9 percent to about 8.0 percent.

As stated above, the mice were sacrificed by CO₂ inhalation and bled from the abdominal vena cava. The aortas were perfusion-fixed and post-fixed in 4 percent paraformaldehyde. Atherosclerosis quantitation was assessed using two independent methods: "*en face*" surface lesion analysis and aortic root analysis. The aortic arch and thoracic aorta (defined as above the last intercostal artery) was subjected to "*en face*" analysis. Briefly, the aorta with major branches (left common carotid artery, right common carotid artery, left subclavian artery) was opened up. The lipid deposition in the inner aortic wall was stained in Sudan IV buffer. Images of the Sudan-IV stained aortas were captured with a video camera, and morphometric imaging carried out using Image-Pro 6.0 analysis software (Media Cybernetics, Inc. Carlsbad, CA). The extent of atherosclerosis is expressed as the percent of the aortic surface area covered by lesions. Aortic root analysis provided a second method verifying atherosclerosis quantitation. Briefly, alternate 8-µm frozen sections covering 300 µm of the proximal aorta, starting at the aortic sinus, were stained for atherosclerotic lesions with oil red O. Images of oil red O-stained aortic root sections were captured and analyzed using Image-Pro 6.0 analysis software.
All samples were coded by one investigator and analyzed by another investigator who is completely blinded to the codes.

The abdominal aorta was subjected to abdominal aortic adventitia lesion analysis. Mice with an increase in aortic diameter greater than 50 percent were considered to have an adventitia lesion. Tissue affected by adventitia lesion was categorized independently by two observers. Classification of the adventitia lesions was determined according to the scale based on the gross appearance of the aorta reported by Daugherty *et al.* Briefly, I: Dilated lumen in the suprarenal region of the aorta with no thrombus; II: Remodeled tissue in the suprarenal region that frequently contains thrombus; III: A pronounced bulbous form of type II that contains thrombus; IV: A form in which there are multiple adventitia lesions containing thrombus, some overlapping, in the suprarenal area of the aorta.

The paraformaldehyde-perfused aortas covering the adventitia lesional area were embedded in paraffin. Sections (1 mm) of the abdominal aortas were stained with Hematoxylin & Eosin (H & E), Verhoeffs and Van Gisson staining for elastin, CD68 (FA-11, Serotec Ltd., Kidlington, U.K.) for macrophage and CD31 (Santa Cruz Biotechnology, Santa Cruz, CA) for endothelial cells. The macrophage composition at the aortic root was analyzed on fresh frozen tissues by immunohistochemistry using anti-CD68 primary antibody. Secondary antibodies were donkey anti rabbit, HRP-conjugated; donkey anti rat, HRP conjugated (Jackson Immunoresearch Laboratories, West Grove, PA).

Plasma triglyceride and total cholesterol levels were determined via an automated enzymatic technique (Equal Diagnostic Inc., Exton, PA). Mouse lipoproteins were separated by fast protein liquid chromatography (FPLC) analysis of plasma using 2 Superose 6 columns in series (Pharmacia Biotech Inc., Piscataway, NJ). Aliquots of mouse plasma (100 µl) were injected onto the column, separated with a buffer containing 0.15 M NaCl, 0.01 M Na₂HPO₄, and 0.1 mM EDTA (pH 7.5) at a flow rate of 0.5 mL/min, and analyzed for lipids.

Initial analyses were performed by the Student's *t* test. If the data did not fit the constraints of this parametric test, data were analyzed with the one-way ANOVA test. *P* <0.05 was considered significant. Prism 4.0 software (GraphPad Software Inc., Lake Forest, CA) was used for all calculations. All data are presented as mean ± SEM.

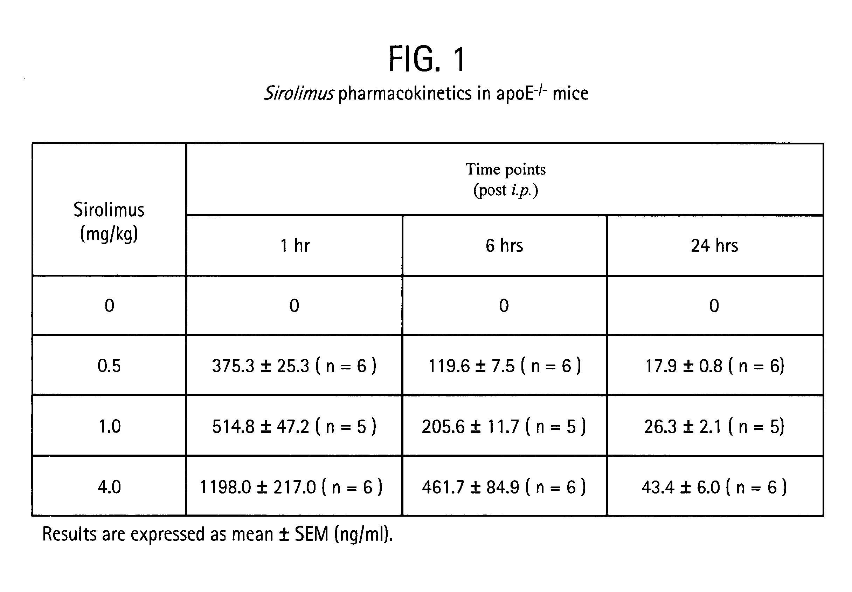

The regimen of sirolimus administration (*i.p.,* daily, 0.5, 1.0, and 4.0 mg/kg) results in significant increase of whole blood sirolimus levels in the apoE^{-/-} mice within 24 hours post injection. The blood drug levels reached peak level at 1 hour post injection, dropped to approximately 30 to 40 percent at 6 hours and remained at trace level at 24 hours. A clear drug dose curve was observed in this whole blood pharmacokinetic study as illustrated by the data in the tabular form (Table 1) illustrated in Figure 1.

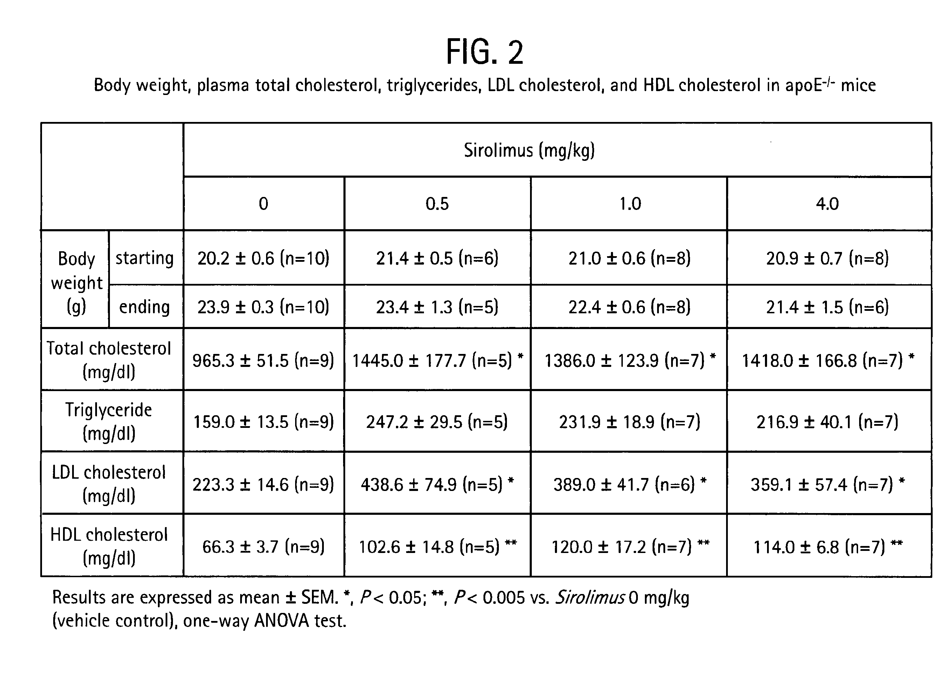

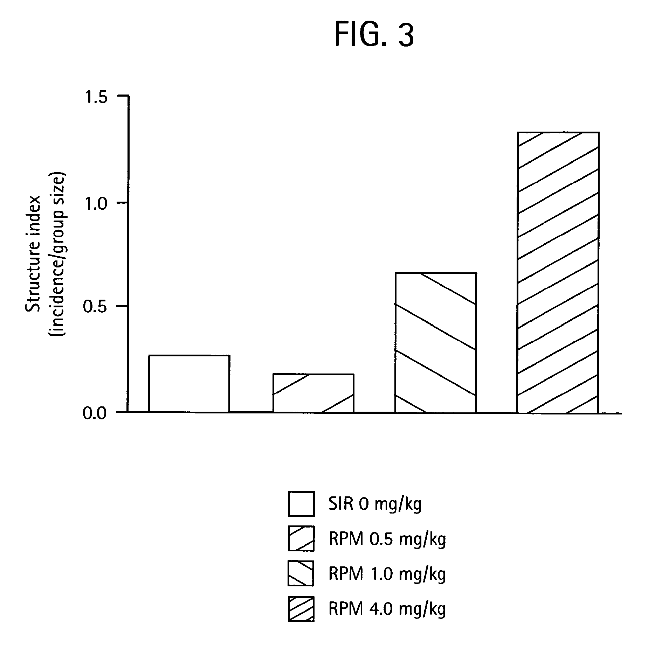

The health status of apoE^{-/-} mice in the current study was not overtly affected by dosing with sirolimus. A trend of decreased body weight was observed in all the 3 sirolimus treated groups but no statistical significance was reached as illustrated by the data in tabular form (Table 2) in Figure 2. sirolimus administration is associated with increased incidence of re-suturing post the surgery for mini-pump implantation in a clear dose dependent manner as illustrated in Figure 3, suggesting a role of sirolimus *in* retarding wound healing in this particular animal model. The data is depicted as re-suturing incedince post surgery per group size. A clear increase of the suture index is observed in response to sirolimus administration in a dose dependent manner.

High fat, high cholesterol diet and angll infusion resulted in marked hyperlipidemia in the vehicle control group, with total cholesterol approaching ~1000 mg/dl (see Table 2 illustrated in Figure 2). Sirolimus administration was associated with about 44 to 50 percent increase of plasma total cholesterol level as compared with the vehicle control group. LDL cholesterol was elevated greater than approximately 60 percent in all sirolimus treated mice. It should be noted that an approximately 55 to 81 percent increase of HDL cholesterol was also observed in the mice receiving sirolimus. In addition, a trend of elevated triglyceride in mice receiving this immunosuppressant was observed, regardless of the dosage employed.

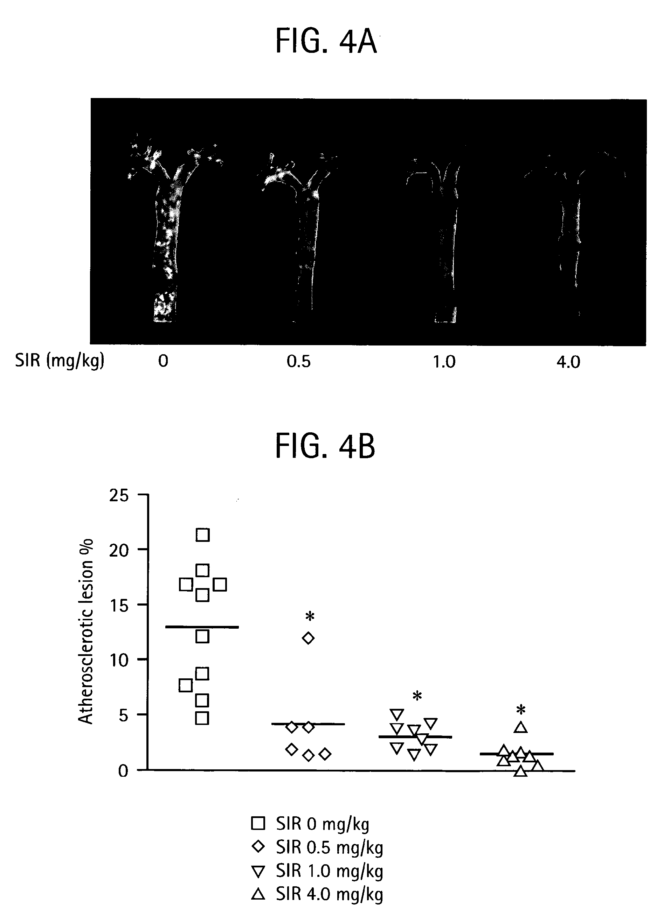

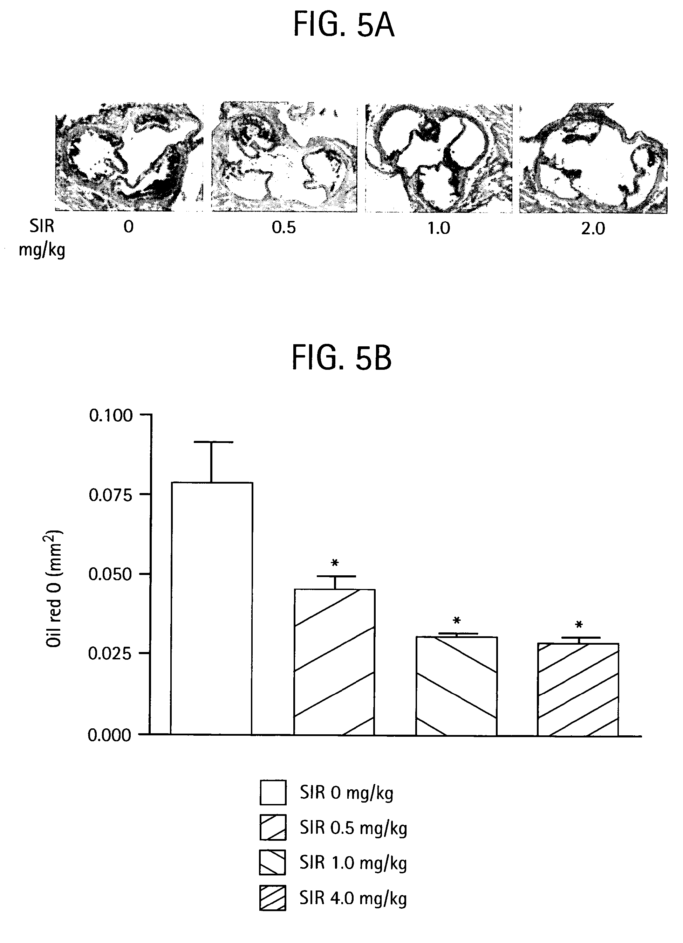

Atherosclerosis quantitation was assessed using two independent methods: *"en face*" analysis and aortic root analysis. Strikingly, at 13 weeks of age in mice infused with angll on a high fat, high cholesterol diet, significantly (*P* < 0.0001, one-way ANOVA) reduced atherosclerotic lesions were seen in mice administrated with sirolimus in a dose dependent manner using *"en face"* analysis as illustrated in Figure 4A and 4B. Figure 4A illustrates a Sudan IV staining of *apoE*^{-/-} mouse aortas with or without simolimus treatment. Each aorta is representative of the mean lesion percentage in the respective groups in Figure 4A, and each point represents percentage lesion even to total aorta area from an individual mouse and bars depict average in Figure 4B.
Significant differences (P< 0.0001, one-way ANOVA) were observed in the sirolimus treated group as compared to the vehicle control groups. This reduction in atherosclerotic lesions was confirmed and extended by aortic root analysis. In this assay, lesion area was significantly (*P* < 0.05, one-way ANOVA) reduced in sirolimus treated groups as compared with vehicle control as illustrated in Figures 5A and 5B. Figure 5A represents oil red O staining of atherosclerotic lesions at the aortic root in *apoE*^{*-*/}*⁻* mice with or without sirolimus treatment. Each section is representative of the mean lesion percentage in the respective groups. Figure 5B represents the quantitative analysis of oil red O positive staining area at the aortic root. Significant differences (*P* < 0.05, one-way ANOVA) were observed in the sirolimus treated groups as compared to the vehicle control groups.

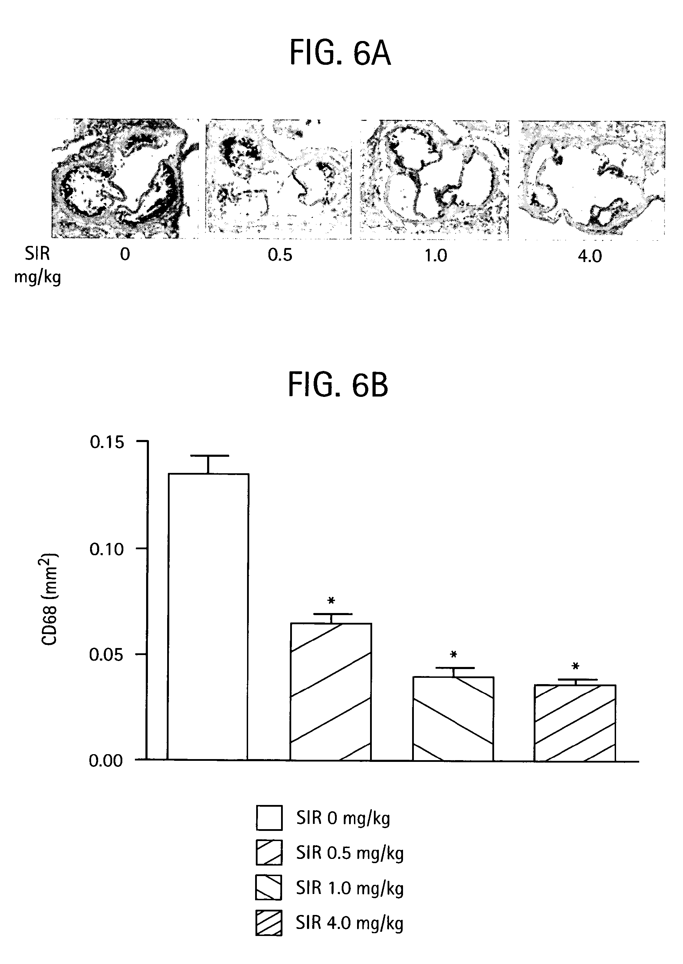

Inflammatory macrophages infiltration into the neo-intima and the subsequent uptake of ox-LDL lead to the formation of foam cells in atherosclerotic lesions. To determine if sirolimus plays an active role in regulating this inflammatory process, immunohistochemical analysis was performed for CD68, a marker of inflammatory macrophages, at the aortic root. Significant reduction in CD68 positive area was clearly apparent in this analysis as illustrated in Figures 6A and 6B. These data suggest that retarded inflammatory macrophage infiltration is responsible for decreased foam cells formation, which in turn leads to reduced lipid deposition in response to sirolimus administration. Figure 6A illustrates immunohistochemical staining for CD68. Sirolimus suppresses CD68 positive signal (brown) in a dose dependent manner. Figure 6B illustrates quantitative analysis of CD68 positive staining at the aortic root. Significant differences (*P* < 0.05, one-way ANOVA) were observed in the sirolimus treated groups as compared to the vehicle control groups.

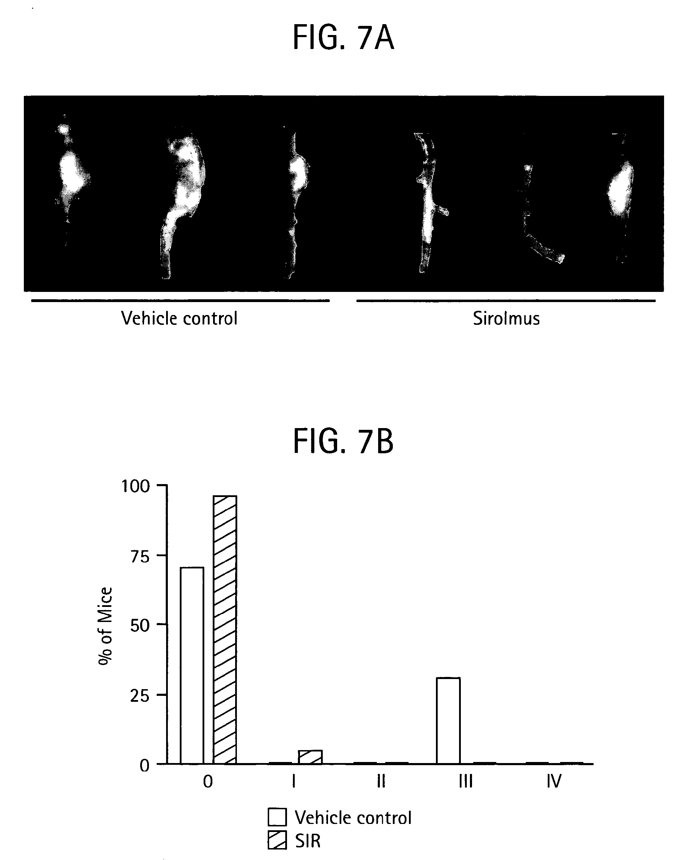

Angll infusion has been demonstrated to induce abdominal aortic adventitia lesion formation in apoE^{-/-} mice on chow diet. In the current methology, angll was infused to apoE^{-/-} mice receiving a high fat, high cholesterol diet. 30 percent incidence of abdominal aortic adventitia lesion was observed in the animals of the vehicle control group. Sirolimus administration exhibited a marked decrease in the incidence of abdominal aortic adventitia lesion (4.5 percent, 1 of 22 mice) compared to vehicle control (30 percent, 3 of 10 mice, abdominal aortic adventitia lesion positive). Representative abdominal aortas from mice with and without sirolimus administration are depicted in Figure 7A. The abdominal aortic adventitia lesions were further classified using previously described criteria described herein. Much less advanced abdominal aortic adventitia lesion was observed in the one positive mouse receiving sirolimus than the ones in the vehicle control group (Figure 7B). Figure 7A illustrates representative abdominal aortas from mice with and without sirolimus. Incidence of abdominal aortic adventitia lesion was reduced in mice receiving sirolimus (4.5 percent) as compared with mice on aortic control (30 percent). Figure 7B illustrates that sirolimus reduced the severity of abdominal aortic adventitia lesions in apoE^{-/-} mice.

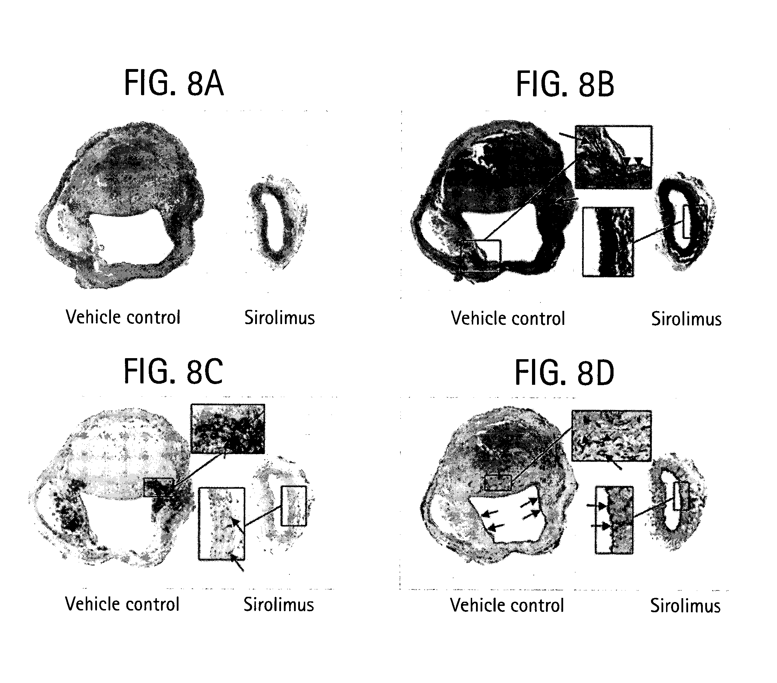

To further characterize the aortic adventitia lesions, histological and immunohistological analyses were performed as illustrated in Figures 8A, 8B, 8C and 8D. Media lamina and elastin disruption has been established as a major pathological process during abdominal aortic adventitia lesion formation. In the vehicle control group, adventitia lesional tissue exhibited complete disruption of media lamina and elastin fibers, while in apoE^{-/-} mice receiving sirolimus, the extent of media disruption was obviously reduced as illustrated in Figure 8B. These data provide the first piece of the mechanism underlying the anti-adventitia lesion capacity of sirolimus *in vivo.* To explore the participation of inflammatory macrophages in media and elastin breakdown, the expression of CD68 was examined, a marker of macrophages, in adventitia lesions induced by angll infusion in apoE^{-/-} mice on a pro-atherogenic diet. Populations of CD68⁺ macrophages were observed in the adventitia lesions. Interestingly, CD68 expression largely co-localized with elastin disruption at the "shoulder area" of the adventitia lesions as illustrated in Figure 8C, suggesting macrophages may play an active role in extracellular matix disruption. Macrophage infiltration into the adventitia lesional tissue was verified using a second marker for macrophage, F4/80 (data not shown). Notably, sirolimus administration prevents inflammatory macrophages accumulation in the granulomas as illustrated in Figure 8C. It has been reported that adventitia lesional tissue contained many *vasa vasorum* in humans, however, as best understood, this has never been documented in mouse models of adventitia lesion formation. The expression of CD31 was explored, an active marker for endothelial cells, in the aortic adventitia lesional tissues. CD31 expression was detected not only in the intima area as expected, but also in the adventitia lesions. CD31⁺ cells preferentially accumulated in the small blood vessel-shape area, indicating *vasa vasorum* in the current mouse model. Sirolimus administration obviously prevents this event in the vessel wall as illustrated in Figure 8D.

## Claims

1. A method of inducing one or more vascular diseases in a mammal comprising:
having at least one of an apoE gene and LDL-receptor gene deleted, the method comprising:
accelerating athereosclorosis in the mammalian vasculature through the introduction of a high fat, high cholesterol diet; and
introducing, via systemic delivery, angiotensin II at a predetermined time to induce and accelerate vascular inflammation.

2. A method of inducing one or more vascular diseases in a mouse comprising:
having at least one of an apoE gene and LDL-receptor gene deleted, the method comprising:
accelerating athereosclorosis in the mammalian vasculature through the introduction of a high fat, high cholesterol diet; and
introducing, via systemic delivery, angiotensin II at a predetermined time to induce and accelerate vascular inflammation.
